# EUROPEAN PATENT SPECIFICATION

(11) **EP 4 203 853 B1**
(45) Date of publication and mention of the grant of the patent: **21.10.2026**
(21) Application number: 21769924.8
(22) Date of filing: 25.08.2021
(51) Int. Cl.: A61F 2/24

(54) **PROSTHETIC VALVE DEVICE FOR TREATMENT OF MITRAL VALVE INSUFFICIENCY**
KLAPPENPROTHESENVORRICHTUNG ZUR BEHANDLUNG VON MITRALKLAPPENINSUFFIZIENZ
DISPOSITIF DE VALVE PROTHÉTIQUE POUR LE TRAITEMENT DE L'INSUFFISANCE DE LA VALVULE MITRALE

(30) Priority: 27.08.2020 DE 102020122386
(43) Date of publication of application: 05.07.2023
(73) Proprietor: MEDIRA GmbH, 72336 Balingen (DE)
(72) Inventor: CENTOLA, Marcos, 72379 Hechingen (DE); SCHAEFFER, Oliver, 78532 Tuttlingen (DE); PISANI, Giuseppe, 72336 Balingen (DE); MOGENTALE, Davide, 72336 Balingen (DE)
(74) Representative: Witte, Weller & Partner Patentanwälte mbB
(86) International application number: PCT/EP2021/073468
(87) International publication number: WO 2022/043365

(56) References cited:
- WO-A1-2013/120082
- WO-A1-2020/157018
- US-A1- 2001 039 450

## Description

The present disclosure relates to a prosthetic valve device for treatment of heart valve diseases, in particular mitral valve insufficiencies, wherein the device comprises: a main body having a lumen and comprising a tubular flexible stent frame having an inner surface and an outer surface, and a prosthetic material covering the stent frame at least partially, and wherein the lumen contains a valve structure. The present disclosure also relates to methods for manufacturing such a device, and methods for treatment.

Diseases of the mitral valve are the second-most common clinically significant form of valvular defect in adults. Mitral valve regurgitation occurs with increasing frequency as part of degenerative changes in the aging process (primary or degenerative Mitral regurgitation) and as consequence of ventricular anatomic changes during myocardium dilatative process (secondary or functional).

The mammalian heart comprises four chambers, i.e. two atria, which are the filling chambers, and two ventricles, which are the pumping chambers. In a mammalian heart, there are four heart valves present which normally allow blood to flow in only one direction through the heart, whereby a heart valve opens or closes depending on the differential blood pressure on each side.

The four main valves in the heart are the mitral valve, representing a bicuspid valve, and the tricuspid valve, which are between the upper atria and the lower ventricles, respectively, and thus are called atrioventricular (AV) valves. Further, there are the aortic valve and the pulmonary valve which are in the arteries leaving the heart. The mitral valve and the aortic valve are in the left heart and the tricuspid valve and the pulmonary valve are in the right heart.

The valves incorporate leaflets or so-called cusps, wherein each valve has three cusps, except for the mitral valve, which only has two.

The mitral and the tricuspid valve are situated, respectively, between the atria and the ventricles and prevent backflow from the ventricles into the atria during systole. They are anchored to the walls of the ventricles by *chordae tendineae* which prevent the valves from inverting. The *chordae tendineae* are attached to papillary muscles that cause tension to better hold the valve. Together, the papillary muscles and the *chordae tendineae* are known as the subvalvular apparatus. While the function of the subvalvular apparatus is to keep the valves from prolapsing into the atria when they close, the subvalvular apparatus, however, has no effect on the opening and closure of the valves, which is caused entirely by the pressure gradient across the valve.

During diastole, a normally-functioning mitral valve opens as a result of increased pressure from the left atrium as it fills with blood (preloading). As atrial pressure increases above that of the left ventricle, the mitral valve opens. Opening facilitates the passive flow of blood into the left ventricle. Diastole ends with atrial contraction, which ejects the final 20% of blood that is transferred from the left atrium to the left ventricle, and the mitral valve closes at the end of atrial contraction to prevent a reversal of blood flow.

Several different kinds of valve disorders are known, such as stenosis, which occurs when a heart valve doesn't fully open due to stiff or fused leaflets preventing them from opening properly, or prolapse, where the valve flaps do not close smoothly or evenly but collapse backwards into the heart chamber they are supposed to be sealing off.

Valve regurgitation (backward flow) is also a common problem, and occurs when a heart valve doesn't close tightly, as a consequence of which the valve does not seal and blood leaks backwards across the valve. This condition - also called valvular insufficiency - reduces the heart's pumping efficiency: When the heart contracts blood is pumped forward in the proper direction but is also forced backwards through the damaged valve. As the leak worsens, the heart has to work harder to make up for the leaky valve and less blood may flow to the rest of the body. Depending on which valve is affected, the condition is called tricuspid regurgitation, pulmonary regurgitation, mitral regurgitation, or aortic regurgitation.

Mitral regurgitation, i.e. the abnormal leaking of blood from the left ventricle through the mitral valve and into the left atrium when the left ventricle contracts, is a common valvular abnormality, being present in 24% of adults with valvular heart disease and in 7% of the population 75 years of age. Surgical intervention is recommended for symptomatic severe mitral regurgitation or asymptomatic severe mitral regurgitation with left ventricular dysfunction or enlargement. Surgical treatment of severe degenerative mitral regurgitation has evolved from mitral valve replacement to mitral valve repair, since a mitral valve repair has proven to produce superior outcomes.

The annual incidence of degenerative Mitral valve disease (primary) in developed nations is estimated at around 2% to 3%. In addition to degenerative changes, secondary Mitral valve regurgitation include cardiac ischemia, infective endocarditis and rheumatic disease, occurs more frequently in less developed countries.

Mitral valve replacement was the highest risk adult cardiac procedure in most centers in the world, with operative mortalities up to 20-30%. Introduction of new techniques, refinement of reparative methods and Carpentier's unified approach bringing together the techniques of ring annuloplasty, leaflet reconstruction, and chordal shortening/transfer with excellent results lead the way to the establishment of Mitral valve repair as the procedure of choice in mitral insufficiency
At least 50% of patients with severe MR are not candidates for surgery due to their age or other co-morbidities.

More recently, transcatheter mitral valve repair has become an alternative way for replacing or supporting a diseased or malfunctioning mitral valve. Percutaneous mitral valve repair technology is currently available for patients with nonsurgical high-risk degenerative mitral regurgitation. Other transcatheter mitral regurgitation repair and replacement technology is at various levels of preclinical and clinical investigation, although the currently tested devices are proving to be more challenging compared to transcatheter aortic valve replacement due to the significantly more complex mitral anatomy and the greater heterogeneity of mitral disease requiring treatment.

Percutaneous mitral valve repair technology is generally based on the same principles as mitral valve surgery: neo-chordae placement, leaflet plication, annuloplasty, papillary modification, and left ventricle remodeling. A different approach not based in surgical experience was proposed some years ago, based on the idea of creating an artificial space between the native leaflets enhancing the coaptation between these leaflets. The device developed for this therapy, named "Spacer" was an endovascular balloon catheter, positioned trans-apically in between the Mitral leaflets with an insufflating port implanted sub-cutaneous allowing controlling the volume of the balloon.

This therapy, while having proven to be efficient in reducing the mitral regurgitation volume, nevertheless, presented unsolved complications, such as thrombogenicity of the balloon-material, which requires anticoagulation therapy to reduce the possibility of strokes; also, the space occupied by the balloon reduces the valve orifice area, which in turn leads to reduction of the perfusion between the atrium and ventricle creating an iatrogenic stenosis in the mitral valve.

In view of the above, there still is the need for a heart valve prosthesis by means of which heart valve regurgitation can be efficiently treated, while at the same time traumatic impact on the heart is minimized.
US 2001/0039450 A1 generally concerns implantable vascular devices and discloses a frame with a covering attached thereto. WO 2013/120082 A1 discloses methods and uses of biological tissues for various stent and other medical applications.

According to the invention, which is defined in appended claims 1 and 14, this and other objects is solved by a prosthetic valve device for treatment of mitral valve insufficiency, wherein the device comprises: a main body having a lumen and comprising a tubular flexible stent frame having an inner surface and an outer surface, and a prosthetic material covering the stent frame at least partially, and wherein the lumen contains a valve structure, wherein the flexible stent frame consists of a plurality of wire-elements, each of the plurality of wire-elements being formed of a single wire having a first wire end portion and a second wire end portion, wherein each of the wire-elements has a proximal wire-element portion and a distal wire-element portion, wherein at the proximal wire-element portion an elongated loop-structure is provided, and wherein the elongated loop-structure comprises a proximal curve section, a distal curve section, and a medium section between the proximal and the distal curve section, wherein the prosthetic material covers the proximal wire-element portion, and in that the prosthetic material is fixed to the proximal curve section from the inner surface of the stent frame, and fixed to the distal curve section from the outer surface of the stent frame.

Preferred embodiments of the invention are defined in the dependent claims.

With the prosthetic valve device of the invention, it is possible to create a spacer for enhancing the coaptation between the native mitral valve leaflets, while at the same time avoiding the related complication of the spacer devices of the prior art.

The prosthetic valve device of the invention can be easily introduced, released and positioned at the native mitral valve region and fixated at the left ventricle apex of the heart of the patient to be treated, without running danger of getting dislocated and injuring the heart tissue. The prosthetic valve device of the invention has the advantage of providing a flexible frame with a tubular segment in the ventricular cavity, leading to a better fatigue resistance. Also, a modular construction with appropriated fixation for the valve leaflets is effected. With the prosthetic valve device of the invention, it is possible to locate the valve of the device in the transition from the left atrium to the left ventricle of a patient's heart, allowing the pressure inside the coaptation cylinder balance to the pressure of native leaflets closure and reducing excessive compress. Also, it the specific attachment of the prosthetic material to the stent frame, a balloon effect of the coaptation cylinder during systolic cycle can be effected, improving coaptation additionally.

The "main body" of the prosthetic valve device comprises or consists of the stent frame, wherein the stent frame is covered by a prosthetic material at least partially. Herein, the expression "at least partially" in view of the prosthesis material means that the stent frame, over a substantial part along its length, is circumferentially covered by the prosthesis material, in particular in the region of the stent frame where the elongated loop-structure/element is provided. Preferably, only the portion of the stent frame carrying the elongated loop-structure/element is covered by the prosthesis material. The wire end portions extend further in the distal/outflow direction and are - over a certain length - not covered by the prosthesis material or contained in any element, so that perfusion inside the wire supported cylindrical body is allowed.

Further, a "valve structure" as herein used, means any element for controlling the passage of blood in one direction only, and in particular includes any artificial or mixed artificial/natural valves or valve tissue resembling a native heart valve of a mammal, in particular a human. In other words, according to the invention, a "valve structure" is a valve element fulfilling functions similar to healthy native heart valves, i.e. allowing blood flow in one direction only.

As used herein, a "wire-element" is an element formed by/through a/one single wire. A "wire", as used and meant herein, as well as known in the prior art, includes any metal/alloy drawn out into the form of a thin flexible longish thread, that has two ends.

In this connection, a wire-element end portion designates a section or area of the end portion of the wire-element located either in the proximally or distally with respect to the inflow end (= proximal/proximally) and the outflow end (= distal/distally).

As used herein, an "elongated loop-structure" means and encompasses any curved shape made when a single wire is bent until one part of it crosses another part of it, thus forming a ring-like structure, wherein the shape of the ring is elongate, i.e. rather oval or substantially oval than circular. According to a preferred embodiment, the elongated loop-structure has a diameter taken in the proximal-distal direction that is larger than a diameter taken in the circumferential direction. The plurality of elongated loop-like elements preferably has the same diameter in proximal-distal direction.

A "curve section" shall designate a curved area/portion of the elongated loop-like element that lies, depending on the definition, proximally or distally, wherein the proximal curve section designates the curve bending in the proximal/inflow direction, and the distal curve section being towards the distal/outflow direction.

According to the invention, a "wire-element" has a loop at the proximal wire-element portion; preferably, the first and second ends, respectively, are comprised by end portions that are not bended or curved, but extend in a straight manner in the distal direction over a certain length. Preferably, the end portions are of the same length.

The expression "proximal", as used herein, generally designates the direction of blood inflow of the prosthetic valve device, i.e. the direction - in relation of the prosthetic valve device - where blood enters to flow from the atrium into the lumen of the prosthetic valve device. Accordingly, the expression "distal" designates the blood outflow direction of the prosthetic valve device, i.e. the end where the blood exits the lumen into the ventricle. Accordingly, the expression "proximal" can also be used synonymously with "inflow" and "proximal" with "outflow".

According to the invention, the prosthetic material is fixed to the stent frame, such, that it covers the proximal wire-element portion carrying the elongated loop-structure. The fixation can be performed/effected by any kind of suitable fixation or attachment means, in particular by sewing, preferably by means of a surgical thread made of PTFE or any other biocompatible material. Preferably, the prosthetic material wherein the prosthetic material does not cover the distal wire-element portion.

According to an embodiment of the prosthetic device of the disclosure, at the proximal wire-element portion an elongated loop-structure is provided and the first and second wire end portions both extend in the distal wire-element portion.

According to preferred embodiments of the disclosure, the loop-structure can be formed by either forming, in the length of a wire, a bend in about the middle of its length, thus generating a proximal bend/curve section and by bringing together, with a half-bend in each of the wire ends, the first and second wire end portions to form a distal wire curve section. Alternatively, according to another preferred embodiment, the proximal and distal curve sections are formed by guiding around a wire-element in a circle.

According to a preferred embodiment of the prosthetic valve device of the disclosure, the loop-structure can be provided as an open or closed loop-structure.

Preferably, when "open", the loop is preferably formed such, that in the length of a wire a bend is formed in about the middle of its length, by generating a proximal bend/curve section and by bringing together the first and second wire end portions to form a distal wire curve section or bend, preferably with each wire forming a half of the distal curve section. In that way, the elongated loop-structure is formed in the proximal wire-element portion, and the first and second wire end portions both extend - as individual end portions - in/towards the distal wire-element portion, i.e. opposite the proximal bend/curve section.

Preferably, in case of a "closed" loop-structure, the first and second wire end portions of the wires opposite the bend/proximal curve are being held together, adjacent and distal to the distal curve section, at least over a certain distance, preferably by a crimp, a sleeve, or by twisting.

According to yet another preferred embodiment, when provided with a closed loop-structure, the first and second wire end portions can be held together by a sleeve substantially over their entire length adjacent to the distal curve section, whereby a joint distal wire-element portion is formed by a joined first and second wire end portion. As a consequence, a first and second wire end portion jointly/as a pair extends in/towards the distal wire-element portion as one unit, respectively.

According to yet another embodiment, the proximal curve section can comprise two layers of the single wire, and the distal curve section comprises one layer of the wire, or in other words, this embodiment is generated by guiding the wire in an oval circle. Also, in this case, the first and the second wire end portions can extend as individual end portions in the distal wire-element portions, or as a joint unit of two ends. In the latter case, the first and second wire end portions of the wires opposite the bend/proximal curve are being held together, adjacent and distal to the distal curve section, at least over a certain distance, preferably by a crimp, a sleeve, or by twisting.

According to the invention, the elongated loop-structure is designed such, that it has a proximal curve section, a distal curve section and a medium section extending between the proximal curve section and the distal curve section, wherein preferably, in the medium section, the wire extends substantially straight. More preferably, the wire extends from the proximal curve section to the medium section into left and right straight wire sections, and further to the distal curve section, wherein the left and right wire sections at the distal curve section each are bend towards each other such, that the distal curve is formed.

In a preferred embodiment, the proximal curve section, with reference to the proximal-distal length axis and/or the lumen, is bent outwardly.

According to another embodiment of the prosthetic device of the disclosure, the wire-elements are attached to one another, preferably via crimp-elements crimping together the wire-elements. In a refinement, the first and second wire ends of one wire element are additionally paired in a sleeve.

According to an refinement of this embodiment, at least one, two, three four, five or six, crimp-elements are provided, preferably adjacent to the proximal curve section, wherein further preferably one crimp-element connects a left and a right wire section of two different wire-elements.

With this embodiment, a more stable connection/assembly of the wire-elements as such can be provided. Accordingly, in case of three wire-elements, three crimp-elements are needed to attach the wire-elements to one another.

According to a refinement of the disclosure, the prosthetic valve device of the disclosure further comprises a tube-element, the tube-element comprising at least one lumen extending through the tube-element, the lumen being designed for accommodating the distal wire-element portion, and preferably for accommodating the first and/or second wire end portions singularly or in pairs.

With the tube-element, the free ends/ end portions of the wire-elements can be guided/threaded or introduced into a lumen, thus avoiding that the free ends can contact/cause injury of the heart tissue. Also, by means of the tube-element, a stable and secure fixation of the wire-elements ends can be achieved, e.g., by apically or septally fixating the tube having "bundled" the wires therein. As such, the "tube"-element represents a shaft for accommodating the wire end portions via which the device can be fixated apically or septally.

According to the disclosure, the tube-element is spaced in a distance from the covered stent frame section; in other words, the tube element is spaced apart from the distal curved section, thus creating a bare stent frame section that contains wires which are not covered and not contained in the tube-element. As mentioned above, this allows perfusion inside the wire supported tubular structure.

According to a preferred embodiment, the plurality of wire-elements consists of at least or exactly three, four, five or six wire-elements. When using three wires, six wire end portions, i.e. two per wire, are extending towards the distal direction, with the loops being at the proximal end, respectively.

According to another refinement, the tube-element has multiple lumens, preferentially radial to the tube section, extending through the tube-element, such, that each of the first and second wire end portions of the single wires is guided through a single lumen of the multiple lumens of the tube-element. According to a preferred embodiment, the tube-element comprises at least or exactly three or six lumens. Three lumens are preferred for the tube-element in cases where there are three wire-elements provided, with the first and second ends of the three wire-elements, respectively, guided in pairs in one lumen, respectively. Six lumens are preferred for the tube-element in cases where there are three wire-elements provided, with the first and second ends of the three wire-elements, respectively, guided separately in a single lumen in the tube-element.

With this embodiment, the wire end portions of the wire-elements can be secured and guided separately or in pairs through the tube-element, thus avoiding friction of the wires against one another.

According to a preferred embodiment, the tube-element has a length that is shorter than the length of the free wire ends/uncovered end portions. Also, the wire ends/end portions, for fixation purposes, can be guided through the lumen(s) so that they exit the lumen(s) of the tube-element so that they can be used for fixating the prosthetic valve device in the heart of the patient to be treated.

According to a preferred embodiment, the tube-element consists of or comprises a material that is selected from any biocompatible polymer or plastic, such as polyether ether ketone (PEEK), polyoxymethylene (POM), polyether (PE), polyamide (PA), polytetrafluoroethylene (PTFE), ceramics, materials of animal or human origin or generally synthetic materials.

According to a preferred embodiment, the prosthetic material is a biocompatible flexible material, and more preferably selected from mammal tissue, PFTE, and is most preferably mammal pericardium.

According to another preferred embodiment, wires consist of a shape-memory alloy, preferably Nitinol.

By using nitinol wires, the elongated loop-structure of the wire-elements can be preformed according to individual or general needs/requirements. I.e., the diameter of the elongated loop-structure, taken in the proximal-distal direction, can be made larger or smaller, and, as a consequence, the tube or covered portion can be longer or shorter in view of the dimensions of the heart/valve to be treated/supported.

According to a preferred embodiment, in the prosthetic valve device of the disclosure the prosthetic material is fixed only to the proximal curve section and the distal curve section.

With this embodiment, the medium section of the prosthetic material is not attached to the stent frame, and as a consequence, can inflate in a balloon-like fashion and can coapt to the native mitral valve during systole.

According to a preferred embodiment of the device of the disclosure, the elongated loop-structure has a substantially oval form.

The present invention also concerns a method for manufacturing a prosthetic valve device, the method comprising the subsequent steps of:
a) providing a plurality of wire elements, preferably three wire-elements, each of the plurality of wire-elements being formed of a single wire having a first wire end portion and a second wire end portion, wherein each of the single wire-elements has a proximal wire-element portion and a distal wire-element portion, wherein at the proximal wire-element portion an elongated loop-structure is provided, and wherein the first and second wire end portions both extend in the distal wire-element portion, wherein each of the elongated loop-structures comprises a proximal curve section and a distal curve section, and a medium section between the proximal and the distal curve section;
b) attaching, preferably sewing, to the proximal curve section a tubular prosthetic material, preferably mammal pericardium, wherein the tubular prosthetic material comprises an inner surface and an outer surface, a first prosthetic material end portion, a second prosthetic material end portion and a medium prosthetic material portion between the first prosthetic material end portion and the second prosthetic material end portion, such, that the wire-elements are attached on the outer surface of the first prosthetic material end portion only, wherein the second prosthetic material end portion and the medium prosthetic material portion remain unattached to the prosthetic material in this attaching step b);
c) subsequently inverting the tubular prosthetic material with the inner surface over the elongated loop-structures at the proximal wire-element portion, such, that the second prosthetic material end portion and the medium prosthetic material portion is folded over the elongated loop-structure towards the distal wire-element portion; and
d) subsequently attaching the second prosthetic material end portion to the distal curve section of the elongated loop-structure, thus forming the prosthetic valve device.

With this method, a cost-effective and efficient way of manufacturing a prosthetic valve device, in particular one as detailed as invention above, can be achieved: By attaching the wire-elements via their elongated loop-structure to the outer surface of the tubular prosthetic material, and by inverting/folding the tubular prosthetic material "inside-out" over the elongated loop-structure, a valve-element is being generated within the lumen of the prosthetic valve device.

According to the method and the prosthetic valve device of the disclosure, the wire-elements, in the first step, are arranged on the outer surface of the tubular prosthetic material, such, that the elongated loop-structures are distributed in circumferential direction at the same heights.

According to a preferred embodiment, in step a), the wire elements are attached to one another by crimp-elements, thus forming the stent frame.

According to a refinement of the method and the prosthetic valve device of the disclosure it is preferred, if the tubular prosthesis material has a first prosthetic material end portion and a second prosthetic material end portion, and that the wire-elements are attached to the outer surface of the tubular prosthesis material, at the first prosthetic material end portion in step b), such, that the proximal curve sections of the wire-elements cover an the first prosthetic material end portion of the tubular prosthesis material, which portion is selected from about a half, a third, a quarter, a fifth of the outer surface of the prosthesis material in step b). While the tubular prosthetic material is attached in the proximal curve section of the elongated loop-structures, its first prosthetic material end portion is fixed in a certain desired distance X from the absolute proximal end of the elongated loop-structures.

According to a preferred embodiment, where three wire-elements are provided, the elongated loop-structures of the wire-elements are dimensioned such, that when arranged in the circumferential direction of the prosthesis material, they abut with each other. In this configuration, the wire-elements, or rather the elongated loop-structures are arranged in a quasi-triangle circumferentially surrounding the prosthetic material in step b).

According to a refinement of the disclosure, i.e. the method and/or the device of the disclosure, the wire-elements can additionally be fixed to one another, e.g. by sewing them together at the respective sites they abut with one another.

According to another refinement, the method further comprises step e): threading the respective first wire end portions and second wire end portions into a lumen of a tube-element, the tube-element comprising at least one lumen extending through the tube-element, the lumen being designed for accommodating the first and second wire ends/ end portions.

In this refinement it is particularly preferred if the wire ends/end portions are introduced into the tube-element such, that the main body carrying the covered section of the stent frame is spaced apart from the tube-element at a certain distance, so that a bare stent frame section is created allowing perfusion.

As already detailed for the prosthetic valve device of the disclosure, and according to a preferred embodiment, the tube-element is a multi-lumen tube-element, comprising at least or exactly three or six lumen for accommodating the first and second wire end portions.

Also, in the method of the disclosure, it is preferred if the wires consist of or contain a shape-memory alloy, preferably Nitinol.

In a step preceding step a) of the method of the disclosure, a step a') can be provided, comprising the generating of an elongated loop-structure in a wire, which can be performed by, e.g., guiding a wire with one end over one pin or two pins that are spaced apart from one another, and heating the wire, thus, generating the elongated loop-structure of the wire-elements.

According to another aspect of the disclosure, a prosthetic valve device is provided that comprises a main body having a cylindrical form and comprising a lumen extending from a main body proximal end to a main body distal end, and a valve at the main body distal end, and that is manufactured by the method as detailed above.

According to yet another aspect of the disclosure, a method for treating a mitral valve disease of a person, preferably a human, in need thereof, is disclosed, the method comprising the steps of providing a prosthetic valve device as detailed above, and implanting the prosthetic valve device in the region of the mitral valve to be treated. Accordingly, the present disclosure also concerns the use of the prosthetic device as described herein for treating a mitral valve disease of a person, preferably a human, in need thereof.

The prosthetic valve device according to the invention can be either surgically implanted or delivered by transcatheter methods. In the latter case, i.e. with a transcatheter method, the device according to the invention is loaded onto a suitable deployment catheter, there being compressed by a retractable sheath or tube or similar. The deployment catheter is inserted into the heart of a patient whose mitral valve needs replacement or support. The tube-element, in this process, is subsequently fixed to the apex in an apical anchor, which grabs and holds the tube in the desired position. The tube-element can be moved in the anchor to the respective best coaptation position and fixed, e.g. via hooks or a plug, in the apex tissue.

When treating the mitral valve trans-apically, the implantation is done with a small surgery to access the apex of the left ventricle, and the deployment catheter having the device according to the invention loaded thereon in a compressed state, is advanced trans-apically into the left ventricle crossing the mitral valve where it is deployed, with the valve-bearing section in the atrial side.

Alternatively, the compressed device can be introduced via the femoral vein or Jugular vein into the right atrium and trans-septally to the left atrium crossing the mitral valve until the catheter tip reaches the left ventricle apex from inside, when it is deployed in order to expand the valve-bearing section in the atrial side. Via a mechanism, the wires are pushed forward and locked out of the tip of the tube-element/catheter, hooking the wire ends into the myocardium. Additionally, the compressed device can be introduced via a small surgical thoracotomy into to the pulmonic vein (right, left, inferior or superior pulmonic vein) to the left atrium where it is deployed in order to expand valve-bearing section in the atrial side.

Upon correct placement, the sheath or the otherwise compressing means is retracted to release the prosthetic valve device of the invention in a stepwise fashion, upon which action the stent frame of the device can expand.

Further advantages and features of the disclosure are set forth in the following description and in the attached figures.

The aforementioned features of the disclosure and the features still to be explained below are shown in the figures, in which:
Fig. 1 shows a schematic drawing of a human heart;
Fig. 2 shows a schematic drawing of an implanted embodiment of the prosthetic valve device of the disclosure, placed in the mitral valve region of a mammal heart;

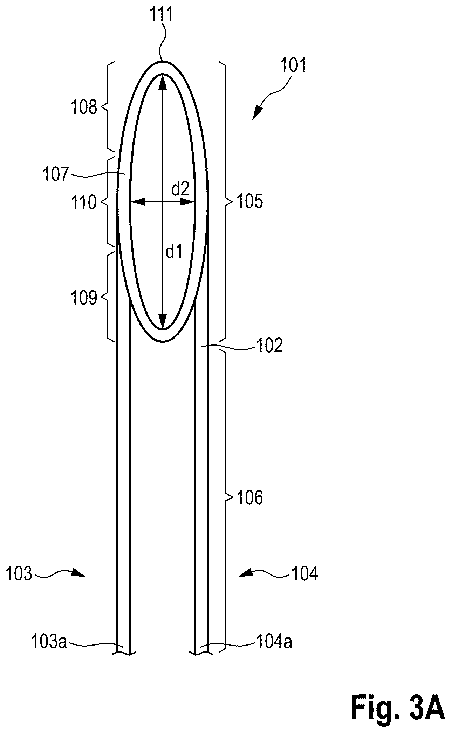

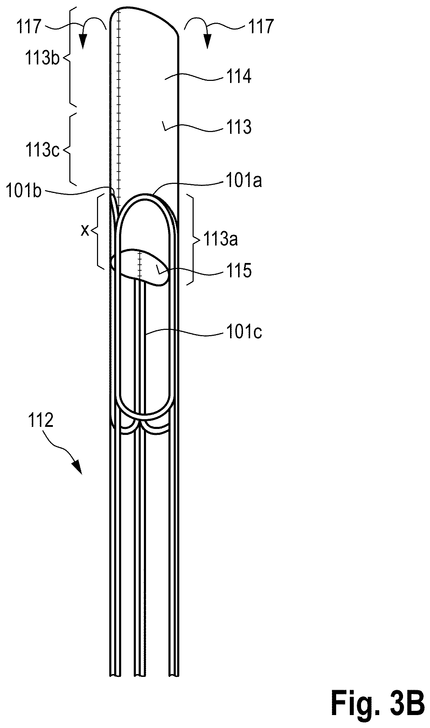

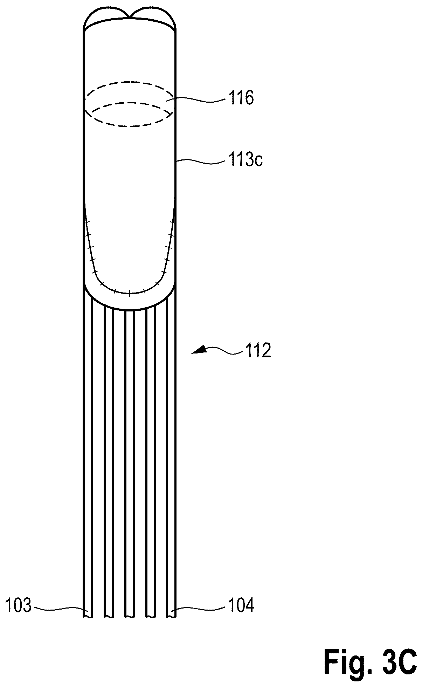

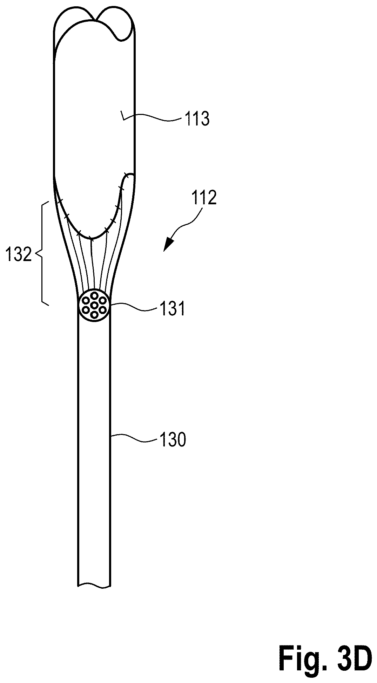

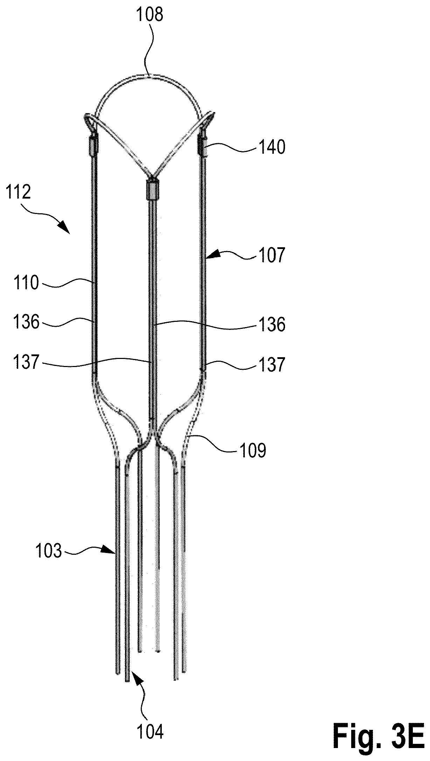

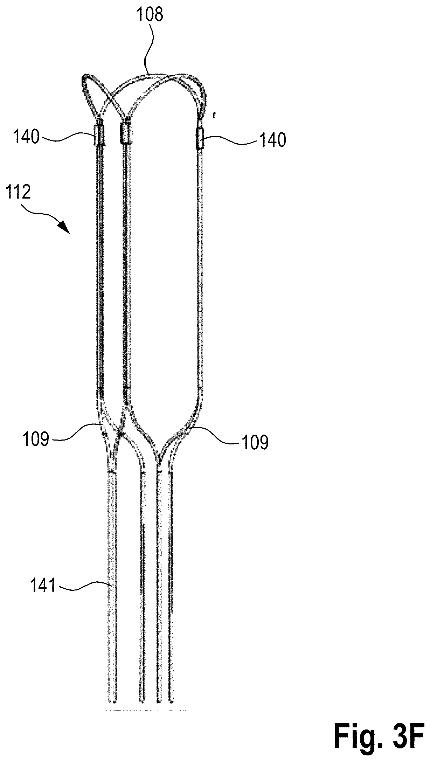

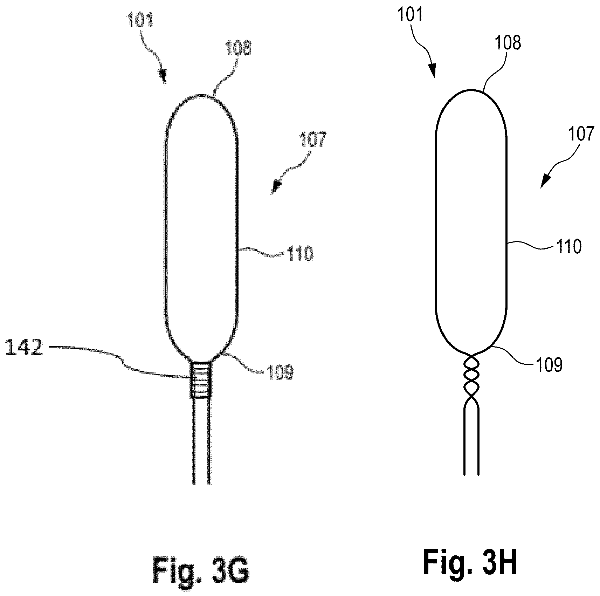

Fig. 3 shows, in schematic drawings in A to D, the steps of manufacturing an embodiment of the prosthetic valve device of the disclosure, not drawn to scale; (A) shows a single wire-element, without having attached thereto the prosthetic material; (B) shows the prosthetic material being attached to the proximal curve section of the elongated loop-structure of three wire-elements; (C) shows the step after folding the tubular prosthetic material inside-out over the elongated loop-structures; (D) shows the threading of the wire end portions into a tube-element, leaving a bare stent frame section and having the tube-element spaced apart from the stent frame section covered by the prosthesis material; with four alternative wire-elements shown in (E), (F), (G) and (H);
Fig. 4 shows two additional embodiments of the prosthetic valve device of the disclosure, with a) showing an embodiment having first fixating means, and b) an embodiment having second fixation means; and
Fig. 5 shows a schematic drawing of another embodiment of the prosthetic valve device of the disclosure, similar to the device as shown in Fig. 3F, with the prosthetic material attached to the stent frame in two different views (A) and (B) and without the prosthetic material (C), and a view from the proximal end into the lumen of the device (D).

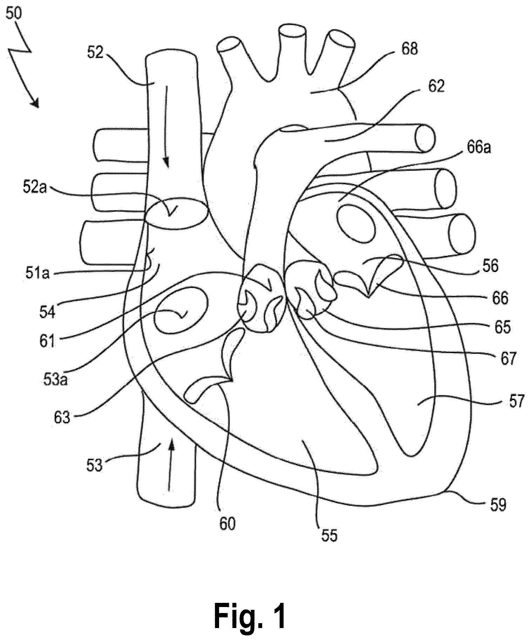

In Fig. 1, a human heart 50 is depicted, having a right atrium 54, a right ventricle 55, a left atrium 56 and a left ventricle 57. Also depicted in fig. 1 is a portion of the vena cava superior 52, entering the heart 50 via the right atrium 54, and a portion of the vena cava inferior 53.

In more detail, the superior vena cava 52 returns the blood from the upper half of the body, and opens into the upper and back part of the right atrium 54, the direction of its orifice 52a being downward and forward. Its orifice 52a has no valve.

The inferior vena cava 53, which has a larger diameter than the superior vena cava 52, returns the blood from the lower half of the body, and opens into the lowest part of the right atrium 54, its orifice 53a being directed upward and backward, and guarded by a rudimentary valve, the valve of the inferior vena cava (Eustachian valve, not shown).

The right ventricle 55 has a triangular in form, and extends from the right atrium 54 to near the apex 59 of the heart 50.

The right atrioventricular orifice (not depicted in fig. 1) is the large oval aperture of communication between the right atrium 54 and ventricle 55, and is guarded by the tricuspid valve 60 comprising three triangular cusps or segments or leaflets 64.

The opening 61 of the pulmonary artery 62 is circular in form, and is placed above and to the left of the atrioventricular opening; it is guarded by the pulmonary valves 63.

As discussed above, the function of the tricuspid valve 60 is to prevent back flow of blood into the right atrium 54; arrows 70 and 71 indicate normal blood flow into the right atrium 54.

The left atrium 56 is smaller than the right atrium 54. The left ventricle 57 is longer and more conical in shape than the right ventricle 55. The left atrioventricular opening (mitral orifice, not depicted in fig. 1) is placed to the left of the aortic orifice 65, and is guarded by the bicuspid or mitral valve 66.

The aortic opening 65 is a circular aperture, in front and to the right of the atrioventricular opening, and its orifice is guarded by the three aortic valves 67. Reference number 68 designates the aorta.

Separating the left atrial chamber or left atrium 56 from the left ventricle 57, the mitral valve 66 is, as mentioned above, an atrio-ventricular valve, with the mitral annulus 70 constituting the anatomical junction between the ventricle 57 and the left atrium 56; the annulus 70 also serves as insertion site for the leaflet tissue (not shown).

The normal mitral valve 66 opens when the left ventricle 57 relaxes (diastole) allowing blood from the left atrium 56 to fill the decompressed left ventricle 57. During systole, i.e. when the left ventricle 57 contracts, the increase in pressure within the ventricle 57 causes the mitral valve 66 to close, preventing blood from leaking into the left atrium 56 and assuring that all of the blood leaving the left ventricle is ejected though the aortic valve 67 into the aorta 68 and to the body. Proper function of the mitral valve is dependent on a complex interplay between the annulus 70, leaflets and subvalvular apparatus (not depicted in Fig. 1, respectively).

Mitral valve 66 regurgitation is present when the valve 66 does not close completely, causing blood to leak back into the left atrium 56.

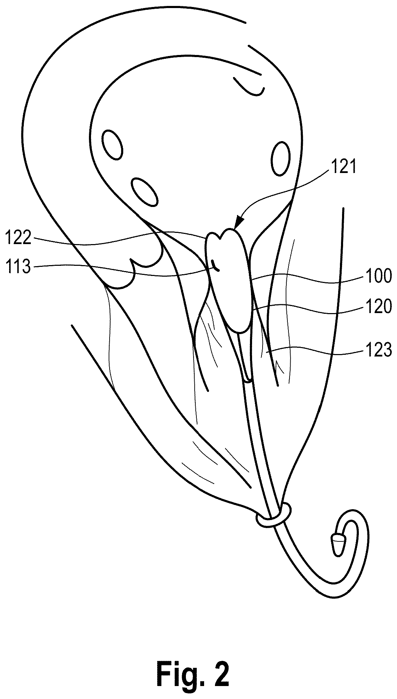

In Fig. 2, an embodiment of a prosthetic valve device 100 of the disclosure is shown, the prosthetic valve device 100 being placed in the heart of a patient. The prosthetic valve device 100 has a main body 120, that has a substantially cylindrical form and comprises a lumen 121 extending from a main body proximal end 122 to a main body distal end 123. The prosthetic valve device 100 further has a stent frame 112 (see, e.g. Fig. 3B) and a prosthetic material 113 attached to the stent frame 112, as well as a valve structure 116 (see Fig.3C). The stent frame 112 has an inner surface 112a and an outer surface 112b.

Fig. 3 shows the elements of an embodiment of the prosthetic valve device 100 in more detail, as well as the manufacturing process of the prosthetic valve device 100 according to the disclosure. In Fig. 3A, a wire-element 101 is shown, formed of a single wire 102 having a first wire end portion 103 and a second wire end portion104, as well as a first wire end 103a and a second wire end 104b. The wire-element 101 has a proximal wire-element portion 105 and a distal wire-element portion 106. As can be seen in Fig. 3A, the wire element 101 has an elongated loop-structure 107 formed in the proximal wire-element portion 105. The elongated loop-structure 107, in this embodiment, is formed by winding the wire once, thus creating an oval loop-structure 107. The loop-structure has a diameter d1, taken proximally to distally, that is larger than the diameter d2 taken circumferentially, thus creating the oval form.

The elongated loop-structure 107 has a proximal curve section 108 and a distal curve section 109, as well as a medium section 110 between the proximal curve section 108 and distal curve section 109.

As can be seen in Fig. 3A, both wire end portions 103 and 104 extend towards the same direction, i.e. the distal/outflow direction, which is opposite to the direction the elongated loop-structure is provided.

Herein, i.e. throughout the disclosure, with the term "wire ends" 103a, 104a the very ends of the wires 102 are designated, wherein with "wire end portions" 103, 104 a section of the respective wire 102 directly adjacent to the specific wire end 103, 104 is designated, although at some passages, when used alternatively, it will be obvious which of the two alternatives, i.e. the very end or an "end portion"/end section is meant.

When manufacturing a prosthetic valve device 100 of the disclosure, at least two, preferably three of the wire-elements 101 are provided, preferably made from nitinol, wherein the elongated loop-structure 107 is generated such, that each of the wire-elements 101 is winded around, e.g., two pins that are spaced from one another in a certain, desired distance, which distance regulates the diameter d1 of the elongated loop-structure 107. Upon heating the wire-elements, they retain the elongated loop-structure 107.

Next, and this can be seen in Fig. 3B, three wire-elements 101a, 101b, 101c are attached two a prosthetic material 113. The prosthetic material 113, as shown in Fig. 3B, is tubular, i.e. formed like a tube, and has an outer surface 114 and an inner surface 115, and a first prosthetic material end portion 113a, and a second prosthetic material end portion 113b. The three wire-elements 101a, 101b and 101c are fixed, preferably sewn, to the outer surface 114 of the prosthetic material 113, at its first prosthetic material end portion 113a such, that the proximal curve section 108 of the elongated loop-structures 107 of the wire-elements 101a, 101b and 101c cover about a third of the prosthetic material 113. As can be seen in the embodiment shown in Fig. 3, the three wire-elements 101a, 101b and 101c are attached by sewing them onto the outer surface 114, such, that they are arranged in a triangle-like fashion circumferentially surrounding the tubular prosthetic material 113 at its first prosthetic material end portion 113a.

In order to generate the valve structure 116, the tubular prosthetic material 113 is inverted, or folded over the proximal curve section 108 of the elongated loop-structures 107, as indicated by the arrows 117 in Fig. 3B, and pulled down over the wire-elements as far as the distal curve sections 109 of the elongated loop-structures 107. In doing so, the inner surface 115 of the tubular prosthetic material 113 gets turned inside-out, and covers the elongated loop-structures 107.

In a next step, as can be seen in Fig. 3C, the second prosthetic material end portion 113b is fixed/attached, preferably sewn to the distal curve sections 109 of the elongated loop-structures 107. In the embodiment shown in Fig. 3, only the second prosthetic material end portion 113b of the tubular prosthetic material 113 is fixed to the elongated loop-structures, so that a medium portion 113c of the tubular prosthetic material 113 remains unattached, allowing the tubular prosthetic material 113 to get inflated in a balloon-like fashion during systole, thus supporting the abutment of the prosthetic valve device 100 to the native mitral valve.

With this step, i.e. inverting or folding the tubular prosthetic material 113 over the proximal curve section 108 of elongated loop-structures 107, a valve structure 116 is generated which is positioned at the attachment site of the first prosthetic material end portion 113a of the prosthetic material 113, which is generally located at the proximal curve section 108, but distanced from the very proximal end 111 of the elongated loop-structures 107 at a distance X (see Fig. 3B). At this distance, now inside the main body 120, the valve structure 116 is generated.

The first and second end portions 103, 104 of the wire-elements, extend the towards the wire-elements distal portion 106.

Fig. 3D shows a last step of assembling a prosthetic valve device 100 according to an embodiment of the invention, where the first and second ends portions 103, 104 of the wire-elements 101a, 101b, 101c are introduced/guided/threaded into a tube-element 130. The tube-element 130, shown in the embodiment in Fig. 3B, has multiple lumens 131. According to this embodiment, the number of lumens 131 corresponds to the number of first and second end portions 103, 104 of the wire-elements 101. As can be also seen in Fig. 3D, the tube-element 130 is placed in a distance of the distal curve of the elongated loop-structures 107, so as to generate a bare stent frame section 132.

In providing the tube-element 130 accommodating the wire-elements 101, it can be advantageously avoided, that the ends/end portions 103, 104, 103a, 104a, cause injuries of the heart tissue. Also, friction of the wire end portions 103, 104, which outwears the wire-elements 101, can be avoided.

Figures 3E, 3F, 3G and 3H show alternative embodiments of the wire-element of the prosthetic device of the disclosure.

As can be seen in the embodiments 3E and 3F, the proximal curve sections 108 are bent outwardly, in respect to longitudinal axis A (see Fig. 3F). Further, as also can be taken from Fig. 3E and 3F, the stent frame 112 is generated such, that three wire-elements 101 are provided, which are connected to one another by crimp-elements 140. The crimp-elements 140 are provided adjacent to the proximal curve section 108, joining left and right wire portions 136, 137 of two different wire-elements 101 together, respectively.

In Fig. 3E, the loop-structures 107 of the wire-elements 101 are formed by a wire 102 being bent in a proximal curve section 108, with a substantially straight medium wire section 110, represented by a left wire portion 136 and a right wire portion 137, respectively, which extend into the distal curve section 109. Towards the distal curve section 109, the left and right wire portions 136, 137, are guided or bend towards one another, thus forming the distal curve section 109. In the distal curve section 109 of the embodiment shown in Fig. 3E, the left and right wire portions 136, 137 are not connected to one another, whereby the generated loop-structure 107 has an "open" loop, with the first and second wire end portions 103, 104 extending separately in the distal wire-element portion 106.

In an alternative embodiment shown in Fig. 3F, which in the general assembly is similar to the one shown in Fig. 3E, the first and second wire end portions 103, 104 are - in pairs - guided/provided in a sleeve 141, distal to/adjacent to the distal curve section 109. As a consequence, the first and second wire end portions 103, 104, will - when the prosthetic device is completely assembled - be guided into the tube-element 130 (not shown in Figs. 3E to 3H) in a pair-wise fashion, i.e. into three lumens 131 of the tube-element 130, while in the embodiment shown in Fig. 3E, six lumens 131 are provided in the tube-element 130 (not shown in Fig. 3E), when threading the first and second wire end portions 103, 104 singularly into the respective lumens 131.

Turning now to Figs. 3G and 3H, yet other embodiments of a wire-element 101 used in the device 100 according to the disclosure are shown. The wire-elements 101 shown in Figs. 3G and 3H, respectively, also comprise a elongated loop-structure 107 having proximal curve section 108, a distal curve section 109, and a medium wire section 110.

While the first and second wire end portions 103, 104, in the embodiment shown in Fig. 3G, are brought together distal to the distal curve section 109 by crimping together via a crimp-structure 142.

Alternatively, and this is shown in Fig. 3H, the first and second wire end portions 103, 104 are - distal to the distal curve section 109 - twisted together.

In both embodiments, the first and second wire end portions 103, 104 can either be separately be guided into a tube-element 103, or in pairs.

In a preferred embodiment, the proximal curve section, with reference to the proximal-distal length axis and/or the lumen, is bent outwardly.

According to another embodiment of the prosthetic device of the disclosure, the wire-elements are attached to one another, preferably via crimp-elements crimping together the wire-elements.

According to an refinement of this embodiment, at least one, two, three four, five or six, crimp-elements are provided, preferably adjacent to the proximal curve section, wherein further preferably one crimp-element connects a left and a right wire section of two different wire-elements.

With this embodiment, a more stable connection/assembly of the wire-elements as such can be provided. Accordingly, in case of three wire-elements, three crimp-elements are needed to attach the wire-elements to one another.

According to a refinement of the disclosure, the prosthetic valve device of the disclosure further comprises a tube-element, the tube-element comprising at least one lumen extending through the tube-element, the lumen being designed for accommodating the distal wire-element portion, and preferably for accommodating the first and/or second wire end portions singularly or in pairs.

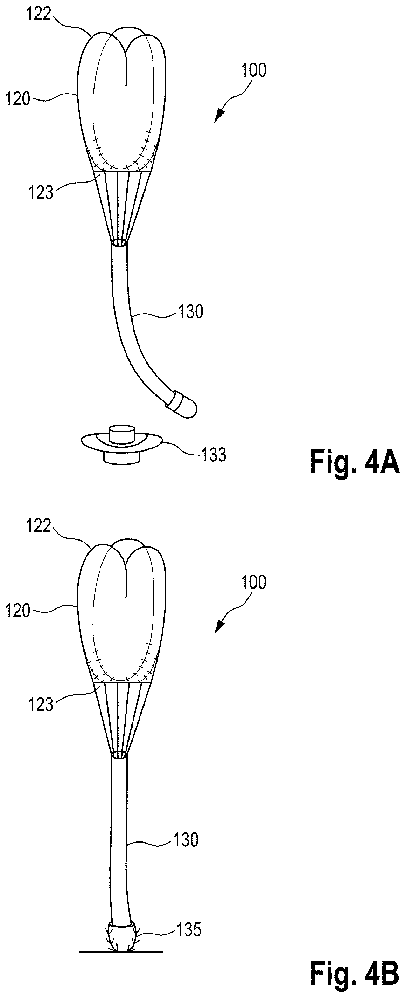

Fig. 4 shows two different possibilities for fixating the prosthetic valve device 100 in the heart of a patient, with Fig. 4A showing an exemplary embodiment that can be used when trans-apically implanting the prosthetic valve device 100 of the disclosure, and Fig. 4B when trans-septically implanting the prosthetic valve device 100 of the disclosure.

The trans-apical implantation is effected by a small surgery to access the apex of the left ventricle and the prosthetic valve device 100 is deployed using a trans-apical catheter (not shown). The tube-element 130 is fixed to the apex in an apical anchor 133 which can grab the tube-element 130 in the desired position. The tube-element 130 can be moved in the anchor 133 to the best coaptation position and fixed, e.g., in a plug-like fashion.

The trans-septal implantation requires a catheter (not shown) that delivers the prosthetic valve device 100 via the femoral vein, right atrium, cross septum, left atrium, crossing mitral valve, until the catheter tip reaches the left ventricle apex from inside. Using a mechanism to slide and lock the wires out of the tube-element the wire-elements can be hooked to the myocardium via hooks 135.

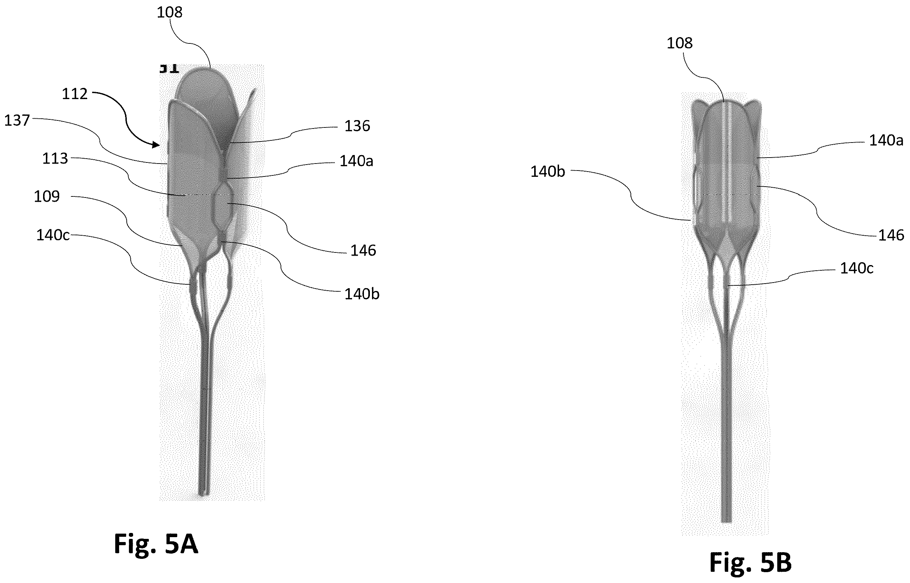

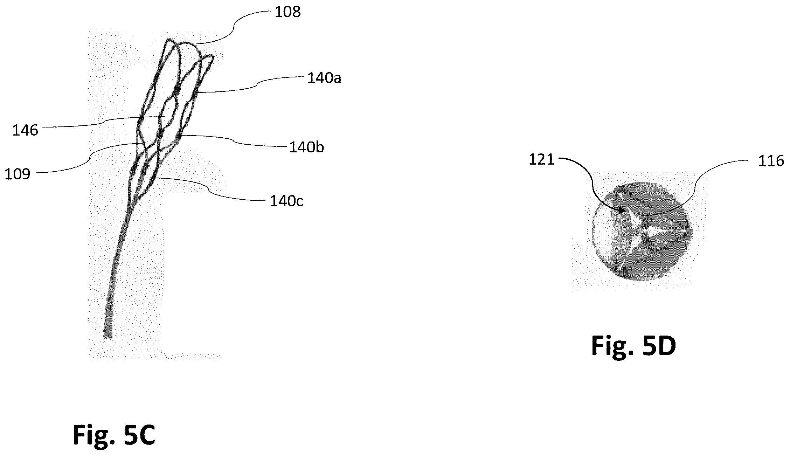

Fig. 5 shows another embodiment of a prosthetic device of the disclosure, which is similar to the device as shown in Fig. 3F, and, accordingly, the same features are designated with the same reference signs. Fig. 5A and 5B each show a side view of the embodiment, with the prosthetic material 113 attached to the stent frame 112. Fig. 5C shows the stent frame 112 of the embodiment without the prosthetic material 113 being attached to the stent frame 112, and Fig. 5D shows a view into the lumen 121 of the device 100 of this embodiment, displaying the valve structure 116.

As can be seen, the proximal curve sections 108 are slightly bent outwardly, in respect to longitudinal axis A. Further, the stent frame 112 is generated such, that three wire-elements 101 are provided, which are connected to one another by crimp-elements 140a, 140b at two different positions, i.e. a more proximal one 140a and a more distal one 140b. The proximal crimp-elements 140a are provided adjacent to the proximal curve section 108, joining left and right wire portions 136, 137 of two different wire-elements 101 together, respectively. The distal crimp-elements 140b are provided proximal to the distal curve section 109, in a certain distance from the proximal crimp-element 104a.

In the embodiment shown in Fig. 5, the two wire elements 101 - or rather their left and right wire portions 136, 137 - crimped together by the proximal and the distal crimp-elements 140a, 140b, form, in between the distance of the proximal and the distal crimp-elements 140a, 104b, a generally oval single-cell-structure 146. Distally of the distal curve section 109, the first and second wire end portions 103, 104 of each of the three wire elements 101 are crimped together by a third crimp-element 140c. Also depicted in Fig 5. is the prosthetic material 113 being fixed to the stent frame 112 with a valve 116 being formed inside the lumen 121 (see Fig. 5C).

## Claims

1. A prosthetic valve device (100) for treatment of mitral valve insufficiency, the prosthetic valve device (100) comprising
a main body (120) having a lumen (121) and comprising a tubular flexible stent frame (112) having an inner surface (112a) and an outer surface (112b), and a prosthetic material (113) covering the stent frame (112) at least partially, and wherein the lumen (121) contains a valve structure (116), wherein
the flexible stent frame (112) consists of a plurality of wire-elements (101), each of the plurality of wire-elements (101) being formed of a single wire (102) having a first wire end portion (103) and a second wire end portion (104), wherein each of the wire-elements (101) has a proximal wire-element portion (105) and a distal wire-element portion (106), wherein at the proximal wire-element portion (105) an elongated loop-structure (107) is provided, the elongated loop-structure (107) comprising a proximal curve section (108) and a distal curve section (109), and a medium section (110) between the proximal (108) and the distal (109) curve section, wherein the prosthetic material (113) covers the proximal wire-element portion, and wherein the prosthetic material (113) is fixed to the proximal curve section (108) from the inner surface (112a) of the stent frame (112), and fixed to the distal curve section from the outer surface of the stent frame (112b), such, that the tubular prosthetic material (113) is, from the inner surface (112a) to the outer surface (112b), folded over the elongated loop-structure (107).

2. The prosthetic valve device (100) of claim 1, further comprising a tube-element (130), the tube-element (130) comprising at least one lumen (131) extending through the tube-element (130), the lumen (131) being designed for accommodating the first and second wire end portions (103, 104).

3. The prosthetic valve device (100) of claim 1 or 2, wherein the plurality of wire-elements (101) consists of three or more wire-elements (101).

4. The prosthetic valve device (100) of any of the preceding claims, wherein the loop-structure (107) is an open or closed loop-structure (107).

5. The prosthetic valve device (100) of any of the preceding claims, wherein the first and second wire end portions (103, 104) of the wires (102) are being held together, adjacent and distal to the distal curve section (109), at least over a certain distance, preferably by a crimp-structure (142) or by twisting the wires (102).

6. The prosthetic valve device (100) of any of the preceding claims, wherein the first and second wire end portions (103, 104) are be held together by a sleeve (141) substantially over their entire length adjacent to the distal curve section (109).

7. The prosthetic valve device (100) of any of the preceding claims, wherein the proximal curve section (108) comprises two layers of the wire (102), and the distal curve section (109) comprises one layer of the wire (102).

8. The prosthetic valve device (100) of any of the preceding claims, wherein crimp-elements (140; 140a, 140b, 140c) are provided connecting the wire-elements (101) with one another.

9. The prosthetic valve device (100) of any of claims 2 to 8, wherein the tube-element (130) has multiple lumens (131) extending through the tube-element (130), such, that each of the first and second wire end portions (103, 104) of the single wires or in pairs (102) is guided through a single lumen of the multiple lumens (131) of the tube-element (130).

10. The prosthetic valve device (100) of any of claims 2 to 9, wherein the tube-element (130) comprises at least or exactly three or six lumens (131).

11. The prosthetic valve device (100) of any of the preceding claims, wherein the wire-elements (101) consist of a shape-memory alloy, preferably Nitinol.

12. The prosthetic valve device (100) of any of the preceding claims, wherein the prosthetic material is fixed only to the proximal curve section and the distal curve section.

13. The prosthetic valve device (100) of any of the preceding claims, wherein the elongated loop-structure (107) has a substantially oval form.

14. Method for manufacturing a prosthetic valve device (100), the method comprising the subsequent steps of:
a) providing a plurality of wire-elements (101), preferably three wire-elements (101), each of the plurality of wire-elements (101) being formed of a single wire (102) having a first wire end portion (103) and a second wire end portion(104), wherein each of the single wire-elements (101) has a proximal wire-element portion (105) and a distal wire-element portion (106), wherein at the proximal wire-element portion (105) an elongated loop-structure (107) is provided, wherein each of the elongated loop-structures (107) comprises a proximal curve section (108) and a distal curve section (109), and a medium section (110) between the proximal (108) and the distal (109) curve section;
b) attaching, preferably sewing, to the proximal curve section (108) a tubular prosthetic material (113), preferably mammal pericardium, wherein the tubular prosthetic material (113) comprises an inner surface (115) and an outer surface (114), a first prosthetic material end portion (113a), a second prosthetic material end portion (113b) and a medium prosthetic material portion (113c) between the first prosthetic material end portion (113a) and the second prosthetic material end portion (113b), such, that the wire-elements (101) are attached on the outer surface (115) of the first prosthetic material end portion (113a) only, wherein the second prosthetic material end portion (113b) and the medium prosthetic material portion (113c) remain unattached to the prosthetic material (113) in this attaching step b);
c) subsequently inverting the tubular prosthetic material (113) with the inner surface (115) over the elongated loop-structures (107) at the proximal wire-element portion (105), such, that the second prosthetic material end portion (113b) and the medium prosthetic material portion (113c) are folded over the elongated loop-structure towards the distal curve section (109); and
d) subsequently attaching the second prosthetic material end portion (113b) to the distal curve section (109) of the elongated loop-structure (107), thus forming the prosthetic valve device (100).

15. The method of claim 14, further comprising step e):
e) threading the respective first wire end portions (103) and second wire end portions (104) into a lumen (131) of a tube-element (130), the tube-element (130) comprising at least one lumen (131) extending through the tube-element (130), the lumen (131) being designed for accommodating the first and second wire end portions (103, 104) either singularly or in pairs in respective lumens (131).

16. The method of claim 14 or 15, wherein the tube-element (130) is a multi-lumen tube-element, comprising at least or exactly three or six lumens (131) for accommodating the first and second wire end portions (103, 104).

## Patentansprüche

1. Prothetische Klappenvorrichtung (100) zur Behandlung einer Mitralklappeninsuffizienz, wobei die prothetische Klappenvorrichtung (100) aufweist:
einen Hauptkörper (120), der ein Lumen (121) aufweist und ein rohrförmiges flexibles Stentgerüst (112) mit einer Innenfläche (112a) und einer Außenfläche (112b) sowie ein das Stentgerüst (112) zumindest teilweise bedeckendes Prothesenmaterial (113) aufweist, wobei das Lumen (121) eine Klappenstruktur (116) enthält, wobei
das flexible Stentgerüst (112) aus einer Vielzahl von Drahtelementen (101) besteht, wobei jedes der Vielzahl von Drahtelementen (101) aus einem einzelnen Draht (102) mit einem ersten Drahtendabschnitt (103) und einem zweiten Drahtendabschnitt (104) gebildet ist, wobei jedes der Drahtelemente (101) einen proximalen Drahtelementabschnitt (105) und einen distalen Drahtelementabschnitt (106) aufweist, wobei an dem proximalen Drahtelementabschnitt (105) eine längliche Schleifenstruktur (107) vorgesehen ist, wobei die längliche Schleifenstruktur (107) einen proximalen Bogenabschnitt (108), einen distalen Bogenabschnitt (109) und einen Mittelabschnitt (110) zwischen dem proximalen (108) und dem distalen (109) Bogenabschnitt aufweist, wobei das Prothesenmaterial (113) den proximalen Drahtelementabschnitt bedeckt,
und wobei
das Prothesenmaterial (113) an dem proximalen Bogenabschnitt (108) von der Innenfläche (112a) des Stentgerüsts (112) her befestigt ist und an dem distalen Bogenabschnitt von der Außenfläche (112b) des Stentgerüsts (112) her befestigt ist, derart, dass das rohrförmige Prothesenmaterial (113) von der Innenfläche (112a) zu der Außenfläche (112b) über die längliche Schleifenstruktur (107) umgefaltet ist.

2. Prothetische Klappenvorrichtung (100) nach Anspruch 1, ferner aufweisend ein Rohrelement (130), wobei das Rohrelement (130) mindestens ein sich durch das Rohrelement (130) erstreckendes Lumen (131) aufweist, wobei das Lumen (131) zur Aufnahme des ersten und des zweiten Drahtendabschnitts (103, 104) ausgebildet ist.

3. Prothetische Klappenvorrichtung (100) nach Anspruch 1 oder 2, wobei die Vielzahl von Drahtelementen (101) aus drei oder mehr Drahtelementen (101) besteht.

4. Prothetische Klappenvorrichtung (100) nach einem der vorhergehenden Ansprüche, wobei die Schleifenstruktur (107) eine offene oder geschlossene Schleifenstruktur (107) ist.

5. Prothetische Klappenvorrichtung (100) nach einem der vorhergehenden Ansprüche, wobei der erste und der zweite Drahtendabschnitt (103, 104) der Drähte (102) benachbart zu und distal von dem distalen Bogenabschnitt (109) zumindest über eine bestimmte Strecke zusammengehalten werden, vorzugsweise mittels einer Crimpstruktur (142) oder durch Verdrillen der Drähte (102).

6. Prothetische Klappenvorrichtung (100) nach einem der vorhergehenden Ansprüche, wobei der erste und der zweite Drahtendabschnitt (103, 104) über im Wesentlichen ihre gesamte, an den distalen Bogenabschnitt (109) angrenzende Länge durch eine Hülse (141) zusammengehalten werden.

7. Prothetische Klappenvorrichtung (100) nach einem der vorhergehenden Ansprüche, wobei der proximale Bogenabschnitt (108) zwei Lagen des Drahts (102) aufweist und der distale Bogenabschnitt (109) eine Lage des Drahts (102) aufweist.

8. Prothetische Klappenvorrichtung (100) nach einem der vorhergehenden Ansprüche, wobei Crimpelemente (140; 140a, 140b, 140c) vorgesehen sind, welche die Drahtelemente (101) miteinander verbinden.

9. Prothetische Klappenvorrichtung (100) nach einem der Ansprüche 2 bis 8, wobei das Rohrelement (130) mehrere sich durch das Rohrelement (130) erstreckende Lumina (131) aufweist, derart, dass jeweils der erste und der zweite Drahtendabschnitt (103, 104) der einzelnen Drähte (102), einzeln oder paarweise, durch jeweils ein Lumen der mehreren Lumina (131) des Rohrelements (130) geführt ist.

10. Prothetische Klappenvorrichtung (100) nach einem der Ansprüche 2 bis 9, wobei das Rohrelement (130) mindestens oder genau drei oder sechs Lumina (131) aufweist.

11. Prothetische Klappenvorrichtung (100) nach einem der vorhergehenden Ansprüche, wobei die Drahtelemente (101) aus einer Formgedächtnislegierung, vorzugsweise Nitinol, bestehen.

12. Prothetische Klappenvorrichtung (100) nach einem der vorhergehenden Ansprüche, wobei das Prothesenmaterial nur an dem proximalen Bogenabschnitt und dem distalen Bogenabschnitt befestigt ist.

13. Prothetische Klappenvorrichtung (100) nach einem der vorhergehenden Ansprüche, wobei die längliche Schleifenstruktur (107) eine im Wesentlichen ovale Form aufweist.

14. Verfahren zur Herstellung einer prothetischen Klappenvorrichtung (100), wobei das Verfahren die folgenden aufeinanderfolgenden Schritte aufweist:
a) Bereitstellen einer Vielzahl von Drahtelementen (101), vorzugsweise drei Drahtelementen (101), wobei jedes der Vielzahl von Drahtelementen (101) aus einem einzelnen Draht (102) mit einem ersten Drahtendabschnitt (103) und einem zweiten Drahtendabschnitt (104) gebildet ist, wobei jedes der einzelnen Drahtelemente (101) einen proximalen Drahtelementabschnitt (105) und einen distalen Drahtelementabschnitt (106) aufweist, wobei an dem proximalen Drahtelementabschnitt (105) eine längliche Schleifenstruktur (107) vorgesehen ist, wobei jede der länglichen Schleifenstrukturen (107) einen proximalen Bogenabschnitt (108), einen distalen Bogenabschnitt (109) und einen Mittelabschnitt (110) zwischen dem proximalen (108) und dem distalen (109) Bogenabschnitt aufweist;
b) Befestigen, vorzugsweise Annähen, eines rohrförmigen Prothesenmaterials (113), vorzugsweise Säugetierperikard, an dem proximalen Bogenabschnitt (108), wobei das rohrförmige Prothesenmaterial (113) eine Innenfläche (115) und eine Außenfläche (114), einen ersten Prothesenmaterial-Endabschnitt (113a), einen zweiten Prothesenmaterial-Endabschnitt (113b) und einen mittleren Prothesenmaterialabschnitt (113c) zwischen dem ersten Prothesenmaterial-Endabschnitt (113a) und dem zweiten Prothesenmaterial-Endabschnitt (113b) aufweist, derart, dass die Drahtelemente (101) ausschließlich an der Außenfläche (115) des ersten Prothesenmaterial-Endabschnitts (113a) befestigt werden, wobei der zweite Prothesenmaterial-Endabschnitt (113b) und der mittlere Prothesenmaterialabschnitt (113c) bei diesem Befestigungsschritt b) unbefestigt an dem Prothesenmaterial (113) bleiben;
c) anschließendes Umstülpen des rohrförmigen Prothesenmaterials (113) mit der Innenfläche (115) über die länglichen Schleifenstrukturen (107) an dem proximalen Drahtelementabschnitt (105), derart, dass der zweite Prothesenmaterial-Endabschnitt (113b) und der mittlere Prothesenmaterialabschnitt (113c) über die längliche Schleifenstruktur (107) in Richtung des distalen Bogenabschnitts (109) umgefaltet werden; und
d) anschließendes Befestigen des zweiten Prothesenmaterial-Endabschnitts (113b) an dem distalen Bogenabschnitt (109) der länglichen Schleifenstruktur (107), wodurch die prothetische Klappenvorrichtung (100) gebildet wird.

15. Verfahren nach Anspruch 14, ferner aufweisend Schritt e):
e) Einfädeln der jeweiligen ersten Drahtendabschnitte (103) und zweiten Drahtendabschnitte (104) in ein Lumen (131) eines Rohrelements (130), wobei das Rohrelement (130) mindestens ein sich durch das Rohrelement (130) erstreckendes Lumen (131) aufweist, wobei das Lumen (131) dazu ausgebildet ist, den ersten und den zweiten Drahtendabschnitt (103, 104) jeweils einzeln oder paarweise in jeweiligen Lumina (131) aufzunehmen.

16. Verfahren nach Anspruch 14 oder 15, wobei das Rohrelement (130) ein Mehrlumen-Rohrelement ist, das mindestens oder genau drei oder sechs Lumina (131) zur Aufnahme des ersten und des zweiten Drahtendabschnitts (103, 104) aufweist.

## Revendications

1. Dispositif de valve prothétique (100) pour le traitement d'une insuffisance de valve mitrale, le dispositif de valve prothétique (100) comprenant
un corps principal (120) ayant une lumière (121) et comprenant un cadre d'endoprothèse (112) tubulaire flexible ayant une surface interne (112a) et une surface externe (112b), et un matériau prothétique (113) couvrant le cadre d'endoprothèse (112) au moins partiellement, et dans lequel la lumière (121) contient une structure de valve (116), dans lequel
le cadre d'endoprothèse (112) flexible est constitué d'une pluralité d'éléments fil (101), chacun parmi la pluralité d'éléments fil (101) étant formé d'un seul fil (102) ayant une première partie d'extrémité de fil (103) et une seconde partie d'extrémité de fil (104), dans lequel chacun des éléments fil (101) a une partie proximale d'élément fil (105) et une partie distale d'élément fil (106), dans lequel au niveau de la partie proximale d'élément fil (105) une structure en boucle (107) allongée est fournie, la structure en boucle (107) allongée comprenant une section de courbe proximale (108) et une section de courbe distale (109), et une section moyenne (110) entre la section de courbe proximale (108) et distale (109), dans lequel le matériau prothétique (113) couvre la partie proximale d'élément fil, et dans lequel le matériau prothétique (113) est fixé à la section de courbe proximale (108) à partir de la surface interne (112a) du cadre d'endoprothèse (112), et fixé à la section de courbe distale à partir de la surface externe du cadre d'endoprothèse (112b), de telle sorte que le matériau prothétique tubulaire (113) est, de la surface interne (112a) à la surface externe (112b), plié au-dessus de la structure en boucle (107) allongée.

2. Dispositif de valve prothétique (100) selon la revendication 1, comprenant en outre un élément tube (130), l'élément tube (130) comprenant au moins une lumière (131) s'étendant à travers l'élément tube (130), la lumière (131) étant conçue pour recevoir les première et seconde parties d'extrémité de fil (103, 104).

3. Dispositif de valve prothétique (100) selon la revendication 1 ou 2, dans lequel la pluralité d'éléments fil (101) est constituée de trois éléments fil (101) ou plus.

4. Dispositif de valve prothétique (100) selon l'une quelconque des revendications précédentes, dans lequel la structure en boucle (107) est une structure en boucle (107) ouverte ou fermée.

5. Dispositif de valve prothétique (100) selon l'une quelconque des revendications précédentes, dans lequel les première et seconde parties d'extrémité de fil (103, 104) des fils (102) sont maintenues ensemble, adjacentes et distales à la section de courbe distale (109), au moins sur une certaine distance, de préférence par une structure de sertissage (142) ou par torsion des fils (102).

6. Dispositif de valve prothétique (100) selon l'une quelconque des revendications précédentes, dans lequel les première et seconde parties d'extrémité de fil (103, 104) sont maintenues ensemble par un manchon (141) sensiblement sur toute leur longueur adjacente à la section de courbe distale (109).

7. Dispositif de valve prothétique (100) selon l'une quelconque des revendications précédentes, dans lequel la section de courbe proximale (108) comprend deux couches du fil (102), et la section de courbe distale (109) comprend une couche du fil (102).

8. Dispositif de valve prothétique (100) selon l'une quelconque des revendications précédentes, dans lequel des éléments de sertissage (140 ; 140a, 140b, 140c) sont fournis reliant les éléments fil (101) les uns aux autres.

9. Dispositif de valve prothétique (100) selon l'une quelconque des revendications 2 à 8, dans lequel l'élément tube (130) a de multiples lumières (131) s'étendant à travers l'élément tube (130), de telle sorte que chacune des première et seconde parties d'extrémité de fil (103, 104) des fils simples ou par paires (102) est guidée à travers une lumière unique des multiples lumières (131) de l'élément tube (130).

10. Dispositif de valve prothétique (100) selon l'une quelconque des revendications 2 à 9, dans lequel l'élément tube (130) comprend au moins ou exactement trois ou six lumières (131).

11. Dispositif de valve prothétique (100) selon l'une quelconque des revendications précédentes, dans lequel les éléments fil (101) sont constitués d'un alliage à mémoire de forme, de préférence du nitinol.

12. Dispositif de valve prothétique (100) selon l'une quelconque des revendications précédentes, dans lequel le matériau prothétique est fixé uniquement à la section de courbe proximale et à la section de courbe distale.

13. Dispositif de valve prothétique (100) selon l'une quelconque des revendications précédentes, dans lequel la structure en boucle (107) allongée a une forme sensiblement ovale.

14. Procédé de fabrication d'un dispositif de valve prothétique (100), le procédé comprenant les étapes suivantes consistant à :
a) fournir une pluralité d'éléments fil (101), de préférence trois éléments fil (101), chacun de la pluralité d'éléments fil (101) étant formé d'un seul fil (102) ayant une première partie d'extrémité de fil (103) et une seconde partie d'extrémité de fil (104), dans lequel chacun des seuls éléments fil (101) a une partie proximale d'élément fil (105) et une partie distale d'élément fil (106), dans lequel au niveau de la partie proximale d'élément fil (105) une structure en boucle (107) allongée est fournie, dans lequel chacune des structures en boucle (107) allongées comprend une section de courbe proximale (108) et une section de courbe distale (109), et une section médiane (110) entre la section de courbe proximale (108) et distale (109) ;
b) attacher, de préférence coudre, à la section de courbe proximale (108) un matériau prothétique tubulaire (113), de préférence du péricarde de mammifère, dans lequel le matériau prothétique tubulaire (113) comprend une surface interne (115) et une surface externe (114), une première partie d'extrémité de matériau prothétique (113a), une seconde partie d'extrémité de matériau prothétique (113b) et une partie d'extrémité de matériau prothétique moyenne (113c) entre la première partie d'extrémité de matériau prothétique (113a) et la seconde partie d'extrémité de matériau prothétique (113b), de telle sorte que les éléments fil (101) sont attachés sur la surface externe (115) de la première partie d'extrémité de matériau prothétique (113a) uniquement, dans lequel la seconde partie d'extrémité de matériau prothétique (113b) et la partie de matériau prothétique moyenne (113c) restent non-attachées au matériau prothétique (113) dans cette étape b) d'attache ;
c) inverser ensuite le matériau prothétique tubulaire (113) avec la surface interne (115) sur les structures en boucle (107) allongées au niveau de la partie proximale d'élément fil (105), de telle sorte que la seconde partie d'extrémité de matériau prothétique (113b) et la partie de matériau prothétique moyenne (113c) sont pliées sur la structure en boucle allongée vers la section de courbe distale (109) ; et
d) attacher ensuite la seconde partie d'extrémité de matériau prothétique (113b) à la section de courbe distale (109) de la structure en boucle (107) allongée, formant ainsi le dispositif de valve prothétique (100).

15. Procédé selon la revendication 14, comprenant en outre l'étape e) :
e) fileter les premières parties d'extrémité de fil (103) et les secondes parties d'extrémité de fil (104) respectives dans une lumière (131) d'un élément tube (130), l'élément tube (130) comprenant au moins une lumière (131) s'étendant à travers l'élément tube (130), la lumière (131) étant conçue pour recevoir les première et seconde parties d'extrémité de fil (103, 104) soit individuellement, soit par paires dans des lumières (131) respectives.

16. Procédé selon la revendication 14 ou 15, dans lequel l'élément tube (130) est un élément tube à lumières multiples, comprenant au moins ou exactement trois ou six lumières (131) destinées recevoir les première et seconde parties d'extrémité de fil (103, 104).
